# EUROPEAN PATENT APPLICATION

(11) **EP 2 412 721 A1**
(43) Date of publication of application: **01.02.2012**
(21) Application number: 10755676.3
(22) Date of filing: 25.03.2010
(51) Int. Cl.: C07K 14/03, C07K 16/08, G01N 33/569

(54) **KOI HERPES VIRUS-SPECIFIC ANTIBODY AND ANTIGEN THEREOF**

(30) Priority: 26.03.2009 JP 2009077737
(71) Applicant: National University Corporation Tokyo University of Marine Science And Technology, Minato-ku Tokyo 108-8477 (JP)
(72) Inventor: AOKI, Takashi, Tokyo 108-8477 (JP); HIRONO, Ikuo, Tokyo 108-8477 (JP)
(74) Representative: Griffin, Philippa Jane
(86) International application number: PCT/JP2010/002120
(87) International publication number: WO 2010/109874

(57) **Abstract**

The present invention provides: a koi herpes virus-specific antigenic peptide that reacts with an anti-koi herpes virus antibody but does not react with an anti-cyprinid herpes virus-1. antibody; an anti-koi herpes virus-specific antibody that is specific for koi herpes virus and does not exhibit cross-reactivity with cyprinid herpes virus-1; and a test or detection kit using the koi herpes virus-specific antigen or the anti-koi herpes virus-specific antibody. A region useful as the major antigen of koi herpes virus (KHV) was found from the koi herpes virus genomic DNA. Moreover, it was confirmed that an antibody directed against this region as an antigen has high detection sensitivity for koi herpes virus (KHV) without exhibiting cross-reactivity with cyprinid herpes virus-1 (CHV).

## Description

### Technical Field

The present invention relates to an anti-koi herpes virus-specific antibody, a koi herpes virus-specific antigenic peptide, a diagnostic kit for koi herpes virus disease, and a detection kit for koi herpes virus.

### Background Art

Koi herpes virus disease is a disease that affects black carp or colored carp by infection with koi herpes virus (KHV). Once developing the disease, black carp or colored carp shows lethargy and loss of appetite but has a few conspicuous external symptoms. Color deterioration, erosion (sore), or the like is seen in the gill. The disease affects juveniles to adult fish and results in high carp mortality.

The occurrence of the koi herpes virus disease was reported for the first time in Israel in May 1998. Thereafter, the disease also occurred two times in the fall of the year and the spring of the next year in Israel and killed approximately 600 tons of carp including those for exportation. The amount of damage totaled more than 4,000,000 dollars. Thereafter, its occurrence was reported worldwide (Israel, UK, Germany, Netherlands, Belgium, USA, Indonesia, and Taiwan) one after another.

Also in Japan, the Ministry of Agriculture, Forestry and Fisheries announced in November 2003 (Heisei 15) that carp suspected to have koi herpes virus disease were identified in Kasumigaura, Ibaraki. Since then, the occurrence of the koi herpes virus disease was reported in regions such as Aomori, Yamanashi, Mie, Okayama, and Miyazaki. The Ministry of Agriculture, Forestry and Fisheries is also making attempts to prevent the spread of this disease, including the identification of the infection route.

When infectious disease caused by virus occurs in the natural world or a farm, the identification of a causative pathogen thereof is exceedingly important for inhibiting the new transmission of the infectious disease or for preventing or treating the infectious disease. For conventionally identifying general bacteria, viruses, mold, or the like, including pathogens of fish, their morphologies, biochemical properties, or biochemical properties based on immunological approaches have been observed. However, with current progress in biochemistry and molecular genetics, such pathogens can also be identified or detected using their chromosomal DNAs or RNAs, constitutive substances, or metabolites.

A PCR method for specifically detecting koi herpes virus in carp tissues by conventional PCR (see Non-patent Documents 1, 2, and 3) and a method for rapidly and highly sensitively detecting koi herpes virus by a LAMP method (loop-mediated isothermal amplification) (see Non-patent Document 4) are known as genetic testing or koi herpes virus gene detection methods targeting koi herpes virus. Moreover, the present inventors have proposed techniques relating to a primer set or probe for koi herpes virus detection which is capable of rapidly detecting the koi herpes virus gene with high sensitivity and high precision (see Patent Document 1).

In addition to genetic testing, immunological or serological assay is sometimes used as means for diagnosing viral infection. Among others, as to the herpes virus family in which koi herpes virus is classified, it has been reported that a host, once affected thereby, is latently infected *in vivo* even after the symptoms of the disease clear up. The genetic testing using PCR or the like fails to identify the source of infection or a history of infection. Therefore, the development of a specific antibody is important. A commercially available antibody against koi herpes virus is, for example, Anti-Koi Herpes virus (KHV) monoclonal antibody (manufactured by AQUATIC Diagnostic Ltd.). Moreover, it has been reported that an ELISA method is effective as a method for examining a history of infection with koi herpes virus (see Non-patent Document 5). However, antigen preparation that involves adsorption onto a solid phase is complicated.

On the other hand, techniques relating to vaccines have been proposed for the purpose of preventing koi herpes virus disease. An injectable vaccine capable of preventing koi herpes virus disease, containing concentrated inactive koi herpes virus or inactivated koi herpes virus-infected cells as an antigen (see Patent Document 2), a liposome vaccine comprising a liposome prepared by sonication in a state where sonicated koi herpes virus and phospholipid coexist in a solution (see Patent Document 3), and a vaccine composition comprising a fusion protein or protein complex consisting of a koi herpes virus-derived antigenic protein fused with the B subunit of a member of the class AB5 bacterial toxin (see Patent Document 4) are known as such techniques. Moreover, the present inventors have proposed a DNA vaccine for carp for inducing protective immunity against koi herpes virus disease using a gene encoding a koi herpes virus glycoprotein or membrane protein (see Patent Document 5). However, no case discloses the major antigen of koi herpes virus.

### Prior Art Documents

### Patent Documents

Patent Document 1: International Publication No. WO 2007/119557
Patent Document 2: Japanese unexamined Patent Application Publication No. 2007-223913
Patent Document 3: International Publication No. WO 2006/090816
Patent Document 4: Japanese unexamined Patent Application Publication (Translation of PCT Application) No. 2008-531478
Patent Document 5: International Publication No. WO 2007/119279

### Non-patent Documents

Non-patent Document 1: Diseases of aquatic organisms, 2002 Mar 11; 48 (2): 101-108
Non-patent Document 2: Journal of Fish Diseases, 2002 25 (3): 171-178
Non-patent Document 3: Fish Pathology, 2005 40: 37-39
Non-patent Document 4: Virology Journal, 2005 2: 83
Non-patent Document 5: Journal of Fish Diseases, 2009 32 (4): 311-320

### Summary of the Invention

### Object to be Solved by the Invention

In addition to koi herpes virus (KHV; also referred to as cyprinid herpes virus-3; CyHV-3), cyprinid herpes virus-1 (CyHV-1 or CHV) which causes viral carp papillomatosis and cyprinid herpes virus-2 (CyHV-2) which causes hematopoietic necrosis in goldfish have been reported as herpes viruses that infect cyprinids (cyprinid herpes viruses; CyHVs). According to the report (St-Hilaire et al., 2009), the koi herpes virus and the cyprinid herpes virus-1 classified in the same CyHV bring about high cross-reactivity therebetween from the serological standpoint. No conventional anti-koi herpes virus antibody could detect only the koi herpes virus with accuracy. Moreover, another problem was low detection sensitivity for the virus. On the other hand, the major antigen of koi herpes virus useful for development of antibody or vaccine against koi herpes virus, ELISA or the like has not been elucidated. An object of the present invention is to provide a koi herpes virus-specific antigenic peptide, an antibody that is specific for koi herpes virus and does not exhibit cross-reactivity with the other CyHVs, particularly, cyprinid herpes virus-1, and a test or detection kit using the antigen or the antibody.

### Means to Solve the Object

The present inventors have conducted diligent studies to attain the object and searched for an epitope region that is specific for koi herpes virus and reacts well with an anti-koi herpes virus antibody, from koi herpes virus DNA library of 85000 clones corresponding to a nucleotide length approximately 70 times the full length of the koi herpes virus genomic DNA. As a result, the present inventors have completed the present invention by finding that: an antibody directed against the region represented by SEQ ID NO: 1 or 3 as an antigen has high detection sensitivity for koi herpes virus without exhibiting cross-reactivity with cyprinid herpes virus-1; the region represented by SEQ ID NO: 1 or 3 is useful as the major antigen of koi herpes virus; and use of such an antibody and an antigen allows accurate diagnosis of koi herpes virus disease.

Specifically, the present invention relates to: (1) a koi herpes virus-specific antigenic peptide consisting of (A) a peptide consisting of the amino acid sequence represented by SEQ ID NO: 1 or 3 or (B) a peptide consisting of an amino acid sequence having a deletion, substitution or addition of one or more amino acids in the amino acid sequence represented by SEQ ID NO: 1 or 3; (2) a koi herpes virus-specific antigenic peptide which is a partial peptide of a peptide consisting of the amino acid sequence represented by SEQ ID NO: 1 or 3 and comprises a koi herpes virus-specific antigenic determinant; (3) an anti-koi herpes virus-specific antibody which is obtained by immunization with the koi herpes virus-specific antigenic peptide according to (1) or (2) described above and specifically recognizes the koi herpes virus-specific antigenic peptide according to (1) or (2) described above; (4) the anti-koi herpes virus-specific antibody according to (3) described above, wherein the antibody is an anti-koi herpes virus-specific polyclonal antibody; (5) the anti-koi herpes virus-specific antibody according to (3) described above, wherein the antibody is an anti-koi herpes virus-specific monoclonal antibody; (6) a hybridoma cell ORF68 (7C6) (NITE ABP-919) producing an anti-koi herpes virus-specific monoclonal antibody; (7) a diagnostic kit for koi herpes virus disease, comprising an antigenic peptide according to (1) or (2) described above; and (8) a detection kit for koi herpes virus, comprising the anti-koi herpes virus-specific antibody according to any one of (3) to (6) described above.

### Effect of the Invention

According to a koi herpes virus-specific antigenic peptide of the present invention, an antibody that is specific for koi herpes virus and does not exhibit cross-reactivity with cyprinid herpes virus-1 can be prepared. Moreover, use of the koi herpes virus-specific antigenic peptide or an antibody against the koi herpes virus-specific antigenic peptide of the present invention allows accurate diagnosis of koi herpes virus disease and accurate detection of koi herpes virus.

### Brief Description of Drawings

[Figure 1] Figure 1 is a diagram showing results of Western blot analysis on the specificity of an anti-KHV-ORF62 mouse polyclonal antibody (Figure 1(A)) and an anti-KHV-ORF68 mouse polyclonal antibody (Figure 1(B)) for koi herpes virus.
[Figure 2] Figure 2 is a diagram showing results of ELISA analysis on the specificity and detection sensitivity of a commercially available anti-KHV monoclonal antibody (Figure 2A)), an anti-KHV-ORF62 mouse polyclonal antibody (Figure 2B)), and an anti-KHV-ORF68 mouse polyclonal antibody (Figure 2C)) for koi herpes virus.
[Figure 3] Figure 3 is a diagram showing results of Western blot analysis on the specificity of an anti-KHV-ORF62 monoclonal antibody for KHV-ORF62 and KHV-ORF68 antigens. In the diagram, M represents a molecular weight marker; A represents the SDS-PAGE of recombinant KHV-ORF62; and Lanes 1 to 12 represent the KHV-ORF62 antigen (Lanes 1 to 6) and the KHV-ORF68 antigen (Lanes 7 to 12) for anti-KHV-ORF62 monoclonal antibodies 2E10, 10D10, 2A1, 4B7, 5G6, and 4F12 in this order.
[Figure 4] Figure 4 is a diagram showing results of Western blot analysis on the specificity of an anti-KHV-ORF68 monoclonal antibody for KHV-ORF68 and KHV-ORF62 antigens. In the diagram, M represents a molecular weight marker; A represents the SDS-PAGE of recombinant KHV-ORF68; and Lanes 1 to 16 represent the KHV-ORF68 antigen (Lanes 1 to 8) and the KHV-ORF62 antigen (Lanes 9 to 16) for anti-KHV-ORF68 monoclonal antibodies 10E6, 7C6, 8D10, 12A2, 1F3, 5G5, 9C7, and 10F9 in this order.
[Figure 5] Figure 5 is a diagram showing results of Western blot analysis on the specificity of an anti-KHV-ORF68 monoclonal antibody 7C6 for CHV, CyHV-2, and KHV. In the diagram, M represents a molecular weight marker.
[Figure 6] Figure 6 is a diagram showing results of analysis by a fluorescent antibody method on the specificity of an anti-KHV-ORF68 monoclonal antibody 7C6 for CHV, CyHV-2, and KHV.

### Mode of Carrying Out the Invention

In the present invention, the term "koi herpes virus" refers to koi herpes virus (KHV), i.e., cyprinid herpes virus-3 (CyHV-3). Moreover, the term "koi herpes virus-specific" refers to reacting with an antigen derived from koi herpes virus or an anti-koi herpes virus antibody but not reacting with antigens derived from the other CyHVs, particularly, cyprinid herpes virus-1, or an anti-cyprinid herpes virus-1 antibody.

A koi herpes virus-specific antigenic peptide of the present invention is any of a peptide consisting of the amino acid sequence represented by SEQ ID NO: 1 in the Sequence Listing (hereinafter, sometimes referred to as a KHV-ORF62 antigen), a peptide consisting of the amino acid sequence represented by SEQ ID NO: 3 in the Sequence Listing (hereinafter, sometimes referred to as a KHV-ORF68 antigen), a peptide consisting of an amino acid sequence having a deletion, substitution or addition of one or more amino acids in the amino acid sequence represented by SEQ ID NO: 1 or 3, and a peptide which is a partial peptide of a peptide consisting of the amino acid sequence represented by SEQ ID NO: 1 or 3 and comprises a koi herpes virus-specific antigenic determinant. The koi herpes virus-specific antigenic peptide of the present invention is not particularly limited as long as it is a peptide that reacts with an anti-koi herpes virus antibody but does not react with an anti-cyprinid herpes virus-1 antibody, and can be used as an antigen to form an anti-koi herpes virus antibody that does not exhibit cross-reactivity with cyprinid herpes virus-1.

The peptide consisting of the amino acid sequence represented by SEQ ID NO: 1 and the peptide consisting of the amino acid sequence represented by SEQ ID NO: 3 according to the present invention are both encoded by a koi herpes virus antigen-encoding region on the genomic DNA of a koi herpes virus TUMST1 (KHV-TUMST1) strain or a koi herpes virus U (KHV-U) strain. The peptide consisting of the amino acid sequence represented by SEQ ID NO: 1 consists of an amino acid sequence that is a portion of a coding region "KHV ORF62" in a protein comprising an OTU-like cysteine protease domain and is rich in low-complexity regions and coiled coil motifs. This peptide is encoded by the nucleotide sequence (SEQ ID NO: 2) of the plus strand at positions 114797 to 116506 in the KHV-U genomic DNA (ACCESSION No. NC_009127). The peptide consisting of the amino acid sequence represented by SEQ ID NO: 3 consists of an amino acid sequence that is a portion of a coding region "KHV ORF68" in a myosin-like protein, comprises an AAA (ATPase associated with a variety of cellular activities) ATPase domain, and is rich in low-complexity regions and coiled coil motifs. This peptide is encoded by the nucleotide sequence (SEQ ID NO: 4) of the minus strand at positions 131963 to 130461 in the KHV-U genomic DNA.

Examples of the peptide consisting of an amino acid sequence having a deletion, substitution or addition of one or more amino acids in the amino acid sequence represented by SEQ ID NO: 1 or 3 can include peptides consisting of an amino acid sequence having a deletion, substitution or addition of any number of amino acids, for example, 1 to 10 amino acids, preferably 1 to 5 amino acids, more preferably 1 to 3 amino acids, for example, 1 amino acid, in the amino acid sequence represented by SEQ ID NO: 1 or 3.

Of the peptides which are a partial peptide of a peptide consisting of the amino acid sequence represented by SEQ ID NO: 1 or 3 and comprise a koi herpes virus-specific antigenic determinant, examples of the partial peptide of the peptide consisting of the amino acid sequence represented by SEQ ID NO: 1 can include a peptide fragment of 6 to 570 amino acid residues consisting of a contiguous amino acid sequence contained in the amino acid sequence represented by SEQ ID NO: 1. Examples of the partial peptide of the peptide consisting of the amino acid sequence represented by SEQ ID NO: 3 can include a peptide fragment of 6 to 501 amino acid residues consisting of a contiguous amino acid sequence contained in the amino acid sequence represented by SEQ ID NO: 3. The partial peptide of the peptide consisting of the amino acid sequence represented by SEQ ID NO: 1 or 3 preferably consists of an amino acid sequence comprising low-complexity regions and/or coiled coil motifs and more preferably consists of a partial peptide whose 80% or more amino acid residues constitute low-complexity regions and/or coiled coil motifs. These partial peptides can be considered to be a koi herpes virus-specific antigenic peptide when each partial peptide is capable of specifically detecting koi herpes virus by preparing an antibody directed against the partial peptide as an antigen according to a standard method and assaying the reaction of this antibody with koi herpes virus or cyprinid herpes virus-1 by immunoassay known in the art such as a Western blot method or ELISA.

Moreover, the koi herpes virus-specific antigenic determinant (epitope) refers to the minimum unit of an amino acid residue population that is recognized by a koi herpes virus-specific antibody but is not recognized by an antibody against cyprinid herpes virus-1, and means an amino acid sequence consisting of 6 to 10 amino acid residues, which is identical to a portion of the amino acid sequence represented by SEQ ID NO: 1 or 3.

A method for obtaining or preparing the koi herpes virus-specific antigenic peptide of the present invention is not particularly limited. The peptide may be isolated from koi herpes virus, chemically synthesized, or prepared by gene recombination. For example, based on the KHV-U genomic DNA (ACCESSION No. NC_009127) sequence, PCR is performed using primers that amplify a region at positions 114797 to 116506 or positions 131963 to 130461 of the sequence, and the obtained DNA fragment can be introduced to a suitable expression system to obtain the koi herpes virus-specific antigenic peptide of the present invention. Alternatively, the DNA fragment can also be introduced to a fusion protein expression system known in the art to express the antigenic peptide as a fusion protein. Moreover, the koi herpes virus-specific antigenic peptide of the present invention can be synthesized according to a chemical synthesis method such as an Fmoc (fluorenylmethyloxycarbonyl) method or a tBoc (t-butyloxycarbonyl) method.

An anti-koi herpes virus-specific antibody of the present invention is not particularly limited as long as it is an antibody that specifically recognizes the koi herpes virus-specific antigenic peptide of the present invention. Examples of the type thereof can include a monoclonal antibody, a polyclonal antibody, and a single-chain antibody. Moreover, the immunoglobulin class of the antibody is not particularly limited and can be selected from any isotype such as IgG, IgM, IgA, IgD, and IgE. The whole antibody or an antibody fragment such as a Fab or F(ab')2 fragment, CDR, a multifunctional antibody, a single-chain antibody (ScFv), and the like can be used as the form of the antibody. The anti-koi herpes virus-specific antibody of the present invention can be prepared as antiserum or a polyclonal antibody, for example, by immunizing an animal with the koi herpes virus-specific antigenic peptide of the present invention or the partial peptide thereof by intravenous, intraperitoneal, or subcutaneous injection and subsequently collecting blood from the immunized animal. Moreover, the anti-koi herpes virus-specific antibody of the present invention can also be prepared as a monoclonal antibody by immunizing a predetermined animal with the koi herpes virus-specific antigenic peptide or the partial peptide thereof by intravenous, intraperitoneal, or subcutaneous injection, subsequently collecting antibody-producing cells such as spleen cells, lymph node cells, or peripheral blood cells from the immunized animal, fusing these cells with tumor cells, and selecting hybridomas using an appropriate selective medium.

In the method for preparing the anti-koi herpes virus-specific antibody of the present invention, examples of the animal to be immunized include mice, rats, guinea pigs, rabbits, goats, sheep, horses, monkeys, chickens, carp, and goldfish. For preparing the monoclonal antibody, mice, rats, or rabbits from which tumor cells can easily be obtained are preferable. A carrier protein such as keyhole limpet hemocyanin, ovalbumin, or bovine serum albumin can be added, if necessary, to the peptide. In the immunization, an adjuvant can be used in combination therewith. The intervals between immunizations, the number of immunizations, and the dose can be selected within the ranges of, for example, 3-day to 4-week intervals between immunizations, 1 to 10 immunizations, and a dose of 100 µg to 100 mg. However, the present invention is not limited to these conditions. Means known in the art such as electroporation or protoplast cell fusion can be used in the cell fusion. For example, a method using a HAT (hypoxanthine/aminopterin/thymidine) medium can be used in the hybridoma selection. The antibody can be collected from a culture solution supernatant by culturing the monoclonal antibody-producing hybridomas in an appropriate low-protein medium such as a MEM medium. In addition, the antibody can be prepared by intraperitoneally injecting the monoclonal antibody-producing hybridomas to an animal from which the tumor cells are derived, and collecting ascitic fluids. Furthermore, the antibody of the present invention can also be purified by a combination of gel filtration, salting out, affinity purification/IgG purification, and so on according to a standard method from the collected serum, culture solution supernatant, or ascitic fluids.

Specific examples of the monoclonal antibody of the present invention can include an anti-KHV-ORF62 mouse monoclonal antibody 4B7, an anti-KHV-ORF62 mouse monoclonal antibody 2A1, an anti-KHV-ORF62 mouse monoclonal antibody 10D10, an anti-KHV-ORF62 mouse monoclonal antibody 2E10, an anti-KHV-ORF62 mouse monoclonal antibody 4F12, an anti-KHV-ORF62 mouse monoclonal antibody 5G6, an anti-KHV-ORF68 mouse monoclonal antibody 1F3, an anti-KHV-ORF68 mouse monoclonal antibody 5G5, an anti-KHV-ORF68 mouse monoclonal antibody 9C7, an anti-KHV-ORF68 mouse monoclonal antibody 10F9, an anti-KHV-ORF68 mouse monoclonal antibody 7C6, an anti-KHV-ORF68 mouse monoclonal antibody 8D10, an anti-KHV-ORF68 mouse monoclonal antibody 12A2, and an anti-KHV-ORF68 mouse monoclonal antibody 10E6. Among them, the anti-KHV-ORF62 mouse monoclonal antibody 10D10 is preferable because it does not exhibit cross-reactivity with a peptide encoded by the KHV-ORF68 sequence. The anti-KHV-ORF68 mouse monoclonal antibody 7C6 and the anti-KHV-ORF68 mouse monoclonal antibody 10E6 are preferable because they do not exhibit cross-reactivity with a peptide encoded by the KHV-ORF62 sequence. Moreover, the anti-KHV-ORF68 mouse monoclonal antibody 7C6 is particularly preferable because it reacts with a KHV-derived antigen but does not react with CHV- and CyHV-2-derived antigens, and is specific for koi herpes virus.

A hybridoma cell ORF68 (7C6) of the present invention is a hybridoma that produces the anti-KHV-ORF68 mouse monoclonal antibody 7C6, which is a koi herpes virus-specific antibody. The hybridoma cell ORF68 (7C6) was deposited as Accession No. NITE ABP-919 on March 24, 2010 with the National Institute of Technology and Evaluation, Patent Microorganisms Depositary (2-5-8 Kazusakamatari Kisarazu, Chiba, Japan).

A diagnostic kit for koi herpes virus disease of the present invention is a kit comprising the koi herpes virus-specific antigenic peptide of the present invention as an antigen. The diagnostic kit for koi herpes virus disease of the present invention is not particularly limited as long as it is a kit wherein an antibody against koi herpes virus in a sample is detected with antigen-antibody reaction with the antibody as an index. Moreover, a detection kit for koi herpes virus of the present invention is a kit comprising an anti-koi herpes virus-specific antibody that specifically recognizes the koi herpes virus-specific antigenic peptide of the present invention. The detection kit for koi herpes virus of the present invention is not particularly limited as long as it is a kit wherein a koi herpes virus antigen in a sample is detected or quantified with antigen-antibody reaction with the antigen as an index. The anti-koi herpes virus-specific antibody contained in the kit is preferably an anti-koi herpes virus-specific monoclonal antibody. For example, the anti-KHV-ORF68 mouse monoclonal antibody 7C6 can be used.

The antigen contained in the diagnostic kit for koi herpes virus disease of the present invention or the antibody contained in the detection kit for koi herpes virus of the present invention can be used in a free state and, in addition, can be immobilized appropriately on a microtiter plate or beads, a test tube, or the like. Examples of the solid phase for immobilization include: synthetic polymers such as polyethylene, polypropylene, polystyrene, polyvinyl chloride, polyacrylamide, and polymethacrylate; and inorganic polymers such as silica gel and glass. Moreover, for the antigen-antibody reaction used as an index, it is only required that this reaction should be confirmed based on the principles of immunoassay known in the art. The antibody of the present invention or a secondary antibody known in the art can be labeled with an enzyme (e.g., alkaline phosphatase or peroxidase), a fluorescent dye, a radioactive material, colloidal gold, or the like, and used as a reagent. Moreover, the kits may further comprise additives known in the art such as a blocking solution, a buffer, a surfactant, a preservative, and a stabilizer.

Examples of the sample for the diagnostic kit for koi herpes virus disease or the detection kit for koi herpes virus of the present invention can include, but not particularly limited to, the gill, skin, tissue (e.g., liver or kidney tissue), and blood of black carp or colored carp.

### Examples

Hereinafter, the present invention will be described specifically with reference to Examples. However, the technical scope of the present invention is not limited to these examples.

### 1-1. Determination of antigenic region

Phage library was prepared from the genomic DNA of a koi herpes virus TUMST1 (KHV-TUMST1) strain and screened for a koi herpes virus antigenic region by a method shown below.

### 1-2. Preparation of KHV genomic DNA

Carp fin-derived KF-1 cells were cultured in ten 75-cm² flasks. At the point in time when the cells became approximately 90% confluent, the cells were inoculated with a koi herpes virus TUMST1 (KHV-TUMST1) strain and cultured at 25°C for approximately 2 weeks until cytopathic effect (CPE) sufficiently appeared. Subsequently, the purification of koi herpes virus and the extraction of the genomic DNA were performed by the following procedures with reference to the method of Gilad et al. (2002). The culture flasks containing the KF-1 cells thus inoculated with koi herpes virus to form CPE were frozen at -80°C for 24 hours or longer, then thawed at room temperature, and centrifuged at 3500 × g at 10°C for 20 minutes to collect a supernatant containing the virus. The supernatant was further centrifuged at 95300 × g at 10°C for 90 minutes. The obtained pellet was suspended in 1 mL of a TNE buffer (50 mM Tris-HCl, 150 mM NaCl, 1 mM EDTA, pH 7.5), homogenized, and then centrifuged through a density gradient with 10-60% sucrose concentrations. After centrifugation at 77000 × g at 10°C for 18 hours, two bands close to the bottom were collected, lysed in a TNE buffer, and centrifuged at 151000 × g at 10°C for 1 hour. The obtained virus pellet was suspended in a TNE buffer to prepare purified KHV.

The purified KHV was treated with proteinase K at 56°C for 3 hours and then inactivated under conditions involving 70°C for 20 minutes. The koi herpes virus genomic DNA was extracted with a phenol/chloroform/isoamyl alcohol (25:24:1) mixed solution. Subsequently, a 0.1-fold volume of 3 M sodium acetate and a 2.5-fold volume of 95% ethanol were added thereto to perform ethanol precipitation. After centrifugation, the pellet was washed with 70% ethanol, dried, and then suspended in a TE buffer to prepare purified genomic DNA.

### 1-3. Screening of phage library

The purified genomic DNA was fragmented by partial digestion using a restriction enzyme *Sau*3AI. The obtained 0.5 to 2 kb DNA fragments were inserted to the *Bam*HI sites of phagemid vectors pBK-CMV (manufactured by STRATAGENE CORP.) to construct gene expression phage library. Subsequently, phage DNA was packaged according to a standard method. *E. coli* was infected with the obtained phages, and plaques were formed on a plate. The formed phages were transferred to a nitrocellulose membrane and reacted with anti-KHV rabbit serum (kindly provided by Professor Fukuda, Fish Pathology Lab., Tokyo University of Marine Science and Technology). From the obtained positive phages, DNA was isolated and analyzed for the nucleotide sequence of the antigen-encoding region in the positive phages by a standard method. As a result, the nucleotide sequences represented by SEQ ID NOs: 2 and 4 were identified, and the major antigen regions of KHV were obtained, which consisted of SEQ ID NO: 1 which was a partial sequence of KHV-ORF62 or SEQ ID NO: 3 which was a partial sequence of KHV-ORF68.

### 1-4. Preparation of recombinant protein (antigen)

A DNA fragment encoding the major antigen region consisting of a portion of KHV-ORF62 or KHV-ORF68 was obtained by PCR amplification from the purified genomic DNA. A *Bam*HI recognition sequence-added PCR primer KHV_62expF (AAGGATCCCATATGGATCAGATTCCCCCCGTCCCAT; SEQ ID NO: 5) and an *Eco*RI recognition sequence-added PCR primer KHV_62expR (TTGAATTCTCACATCGCGGTGGCGTCAAACTT; SEQ ID NO: 6) were used in the amplification of the KHV-ORF62 DNA fragment. A *Bam*HI recognition sequence-added PCR primer KHV_68expF (AAGGATCCCATATGGATCAGTTCAAGCAGACCACGG; SEQ ID NO: 7) and an *Eco*RI recognition sequence-added PCR primer KHV_68expR (TTGAATTCTCACTGCGACTCGAGCCTGGAGTT; SEQ ID NO: 8) were used in the amplification of the KHV-ORF68 DNA fragment.

Each obtained amplified DNA fragment was inserted to between the *Bam*HI and *Eco*RI sites of expression vectors pET-28 (manufactured by Novagen) to prepare recombinant plasmid vectors, which were designated as pET-KHVORF62 and pET-KHVORF68, respectively. Subsequently, *E. coli* BL21 strains were transformed with pET-KHVORF62 or pET-KHVORF68 and shake-cultured at 37°C in 1 L of 2 × YT medium (1.6% Bacto Tryptone, 1.0% yeast extract, 0.5% NaCl). At the point in time when OD₆₀₀ reached 0.8, IPTG was added thereto at a final concentration of 1 mM to induce recombinant protein expression. The bacterial cells were shake-cultured for another 4 hours to produce recombinant proteins (antigens). An inclusion body, which was a high degree of recombinant protein aggregates, was formed in the bacterial cells.

The *E. coli* BL21 strains thus cultured were collected and ultrasonically disrupted to collect recombinant proteins contained in the insoluble fraction of the inclusion body. The obtained recombinant proteins were purified using Ni-NTA agarose (nickel-nitrilotriacetic acid, manufactured by Qiagen). The purified recombinant proteins had a size of 60 kDa (recombinant protein obtained using pET-KHVORF62) and a size of 56 kDa (recombinant protein obtained using pET-KHVORF68).

2-1. Preparation of mouse polyclonal antibody
Each of two types of the purified recombinant proteins was intraperitoneally inoculated as an antigen (KHV-ORF62 antigen or KHV-ORF68 antigen) in amounts of 100 µg once, 150 µg twice, and 200 µg three times (a total of 6 times) to each mouse (ICR lineage, 5-week-old) for immunization. 62 days later, blood was collected from the mice and centrifuged at 5000 rpm at 4°C for 10 minutes to obtain serum. The obtained serum was used as an anti-KHV-ORF62 mouse polyclonal antibody and an anti-KHV-ORF68 mouse polyclonal antibody in the subsequent analysis.

### 2-2. Confirmation of specificity of polyclonal antibody by Western blot

The obtained anti-KHV-ORF62 mouse polyclonal antibody and anti-KHV-ORF68 mouse polyclonal antibody were confirmed for their antigen specificity by Western blot analysis.

According to the culture method described above, a koi herpes virus TUMST1 (KHV-TUMST1) strain and cyprinid herpes virus-1 (CHV) were separately inoculated to KF-1 cells, which were cultured at 25°C for approximately 2 weeks, then collected, and lysed in a lysis buffer (10 mM Tris-HCl (pH 7.5), 10 mM NaCl, 0.5% NP-40). Subsequently, after centrifugation, the obtained soluble fractions were collected to obtain a KHV-infected KF-1 cell lysate and a CHV-infected KF-1 cell lysate. These cell lysates were subjected to the following Western blotting.

The KHV-infected KF-1 cell lysate, the CHV-infected KF-1 cell lysate, and an uninfected KF-1 cell lysate as a control were subjected to SDS-PAGE (50 µg of each protein per lane) on a 10% acrylamide gel, and the proteins were then blotted onto a polyvinylidene fluoride (PVDF) membrane. Subsequently, the PVDF membrane was blocked for 45 minutes using 3% skimmed milk. The anti-KHV-ORF62 mouse polyclonal antibody and the anti-KHV-ORF68 mouse polyclonal antibody diluted 10000-fold with a TBS buffer were separately added to the membrane and shaken at room temperature for 2 hours.

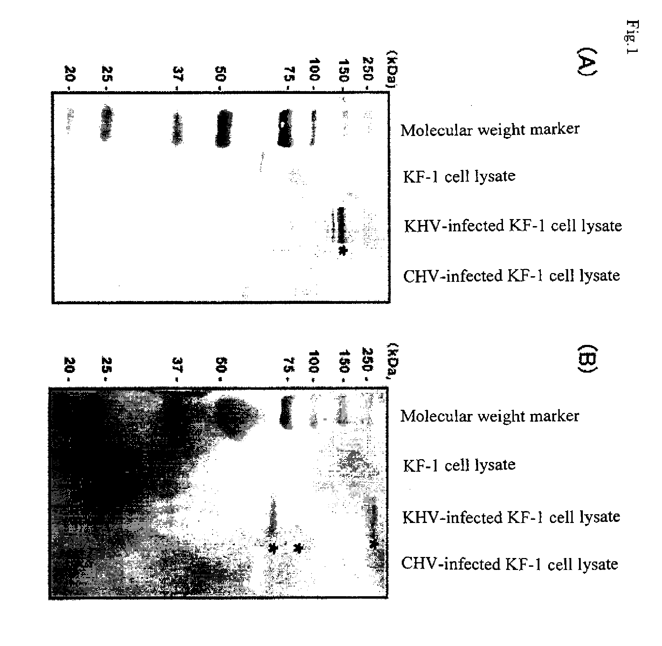

Each polyclonal antibody was detected using an alkaline phosphatase-labeled anti-mouse IgG goat monoclonal antibody diluted 10000-fold as a secondary antibody. The results are shown in Figure 1. As a result of analysis on the anti-KHV-ORF62 mouse polyclonal antibody, approximately 150 kDa proteins were detected in the KHV-infected KF-1 cell lysate (Figure 1A). Moreover, as a result of analysis on the anti-KHV-ORF68 mouse polyclonal antibody, approximately 250 kDa proteins probably encoded by the ORF68 sequence and 90 kDa and 70 kDa proteins probably formed by the processing of the proteins were detected in the KHV-infected KF-1 cell lysate (Figure 1B). By contrast, in any case using the anti-KHV-ORF62 mouse polyclonal antibody and the anti-KHV-ORF62 mouse polyclonal antibody, no protein was detected in the uninfected KF-1 cell lysate and the CHV-infected KF-1 cell lysate. Thus, each polyclonal antibody did not exhibit cross-reactivity.

### 2-3. Confirmation of specificity of polyclonal antibody by ELISA

The obtained anti-KHV-ORF62 mouse polyclonal antibody and anti-KHV-ORF68 mouse polyclonal antibody were confirmed for their antigen specificity by ELISA.

In the same way as above, KF-1 cells infected separately with a koi herpes virus TUMST1 (KHV-TUMST1) strain or cyprinid herpes virus-1 (CHV) were cultured and then lysed in a lysis buffer (10 mM Tris-HCl (pH 7.5), 10 mM NaCl, 0.5% NP-40). A 96-well microtiter plate was coated with the KHV-infected KF-1 cell lysate, the CHV-infected KF-1 cell lysate, or an uninfected KF-1 cell lysate as a control diluted with PBS to 20 µg/mL in terms of a protein concentration. The plate was blocked with 5% BSA to prepare an ELISA plate. A commercially available anti-KHV monoclonal antibody (manufactured by AQUATIC Diagnostic Ltd.) as a Comparative Example, the anti-KHV-ORF62 mouse polyclonal antibody, and the anti-KHV-ORF68 mouse polyclonal antibody were each diluted to 1:500, 1:1000, 1:2000, 1:4000, and 1:8000 and reacted with the prepared ELISA plate for 2 hours.

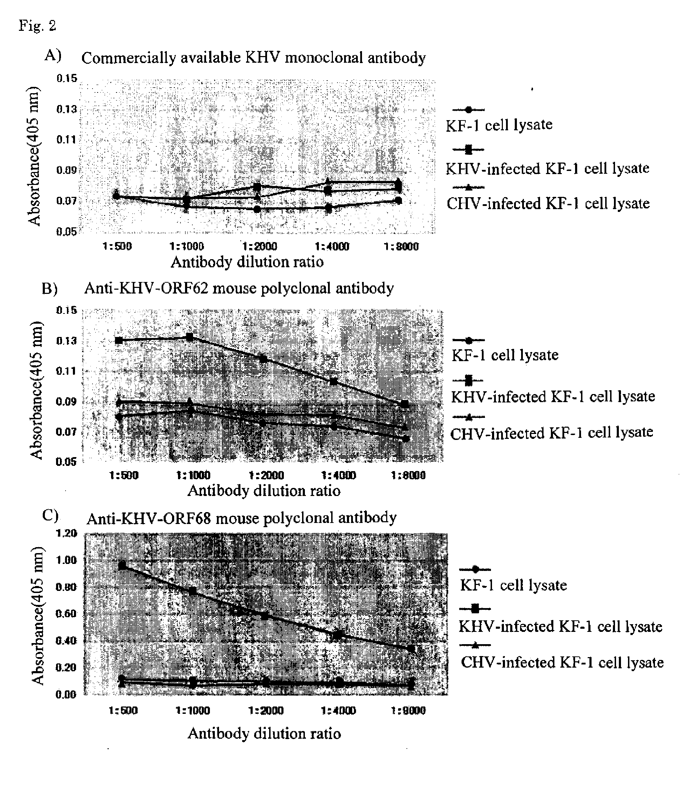

After the reaction, the antibody titers were assayed using SensoLyte^{™} pNPP Alkaline Phosphatase ELISA Assay Kit (manufactured by ANASPEC, Inc.). The absorbance was measured at 405 nm. The results are shown in Figure 2. The commercially available anti-KHV monoclonal antibody did not offer detection sensitivity even when diluted at the lowest ratio of 500 times (Figure 2A). By contrast, the anti-KHV-ORF62 mouse polyclonal antibody and the anti-KHV-ORF68 mouse polyclonal antibody produced sufficient detection sensitivity even when diluted 8000-fold, and no cross-reactivity with the CHV antigen was observed (Figures 2B and 2C).

### 3-1. Preparation of monoclonal antibody

An anti-KHV monoclonal antibody was prepared using, as an antigen, a peptide encoded as the major antigen region consisting of a portion of KHV-ORF62 or KHV-ORF68. Spleen cells were prepared by extracting the spleen from mice (ICR lineage, 5-week-old) immunized with the peptide encoded as the major antigen region consisting of a portion of KHV-ORF62 or KHV-ORF68, separating spleen cells, and washing the cells with PBS. The obtained spleen cells were mixed with a myeloma cell strain SP2. After centrifugation, the supernatant was removed. Cell fusion was performed by a PEG method. The cells were diluted to a predetermined concentration by suspension in an RPMI1640 medium containing 20% FBS, inoculated to a 96-well microtiter plate, and cultured for 7 to 10 days in a CO₂ incubator to prepare hybridomas. The obtained hybridomas were screened by ELISA using KHV antigenic peptides. A 96-well microtiter plate was coated with the KHV-ORF62 antigen or the KHV-ORF68 antigen and blocked with 5% BSA to prepare an ELISA plate. The culture supernatants of the hybridomas were added to each well of the plate and reacted at room temperature (approximately 23°C) for 1 to 2 hours. In ELISA, the antibody titer of a monoclonal antibody produced by each hybridoma was assayed using SensoLyte^{™} pNPP Alkaline Phosphatase ELISA Assay Kit (manufactured by ANASPEC, Inc.). As a result, 6 hybridomas producing the anti-KHV-ORF62 mouse monoclonal antibody were selected: a hybridoma 4B7, a hybridoma 2A1, a hybridoma 10D10, a hybridoma 2E10, a hybridoma 4F12, and a hybridoma 5G6. 8 hybridomas producing the anti-KHV-ORF68 mouse monoclonal antibody were selected: a hybridoma 1F3. a hybridoma 5G5, a hybridoma 9C7, a hybridoma 10F9, a hybridoma 7C6, a hybridoma 8D10, a hybridoma 12A2, and a hybridoma 10E6.

### 3-2. Confirmation of cross-reactivity of monoclonal antibody

The monoclonal antibody produced by each obtained hybridoma was determined for its cross-reactivity with KHV-ORF62 or KHV-ORF68 based on the antibody titer of ELISA. The antibody titer of each anti-KHV-ORF62 mouse monoclonal antibody obtained by immunization with the KHV-ORF62 antigen, for the KHV-ORF62 antigen and the KHV-ORF68 antigen, is shown in Table 1. The antibody titers of each anti-KHV-ORF68 mouse monoclonal antibody obtained by immunization with the KHV-ORF68 antigen, for the KHV-ORF68 antigen and the KHV-ORF62 antigen, are shown in Table 2.

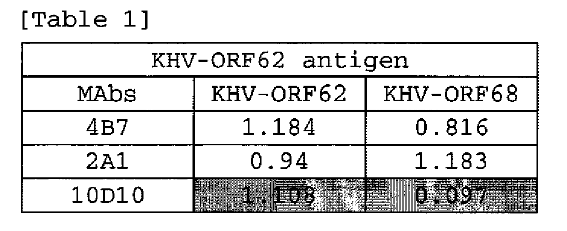

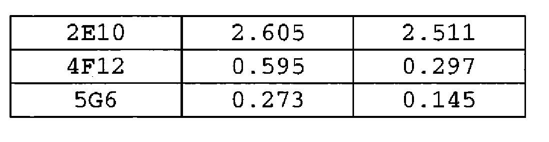

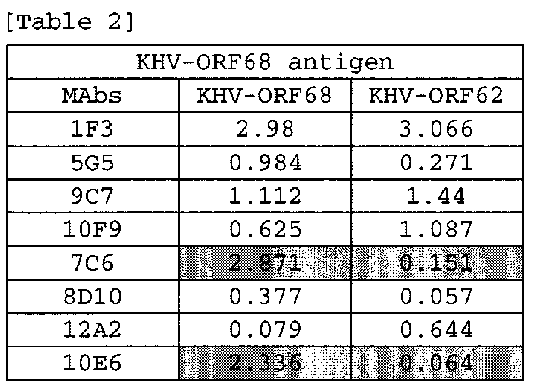

As a result, the anti-KHV-ORF62 mouse monoclonal antibody 10D10 was considered a monoclonal antibody that reacted with the KHV-ORF62 antigen but did not react with the KHV-ORF68 antigen, without exhibiting cross-reactivity. Moreover, the anti-KHV-ORF68 mouse monoclonal antibodies 7C6 and 10E6 were considered monoclonal antibodies that reacted with the KHV-ORF68 antigen but did not react with the KHV-ORF62 antigen, without exhibiting cross-reactivity.

### 3-3. Confirmation of isotype of monoclonal antibody

The isotypes of the obtained monoclonal antibodies were determined. The ELISA values of the anti-KHV-ORF62 mouse monoclonal antibody and the anti-KHV-ORF68 mouse monoclonal antibody, compared with each isotype-specific antibody, are shown in Tables 3 and 4, respectively.

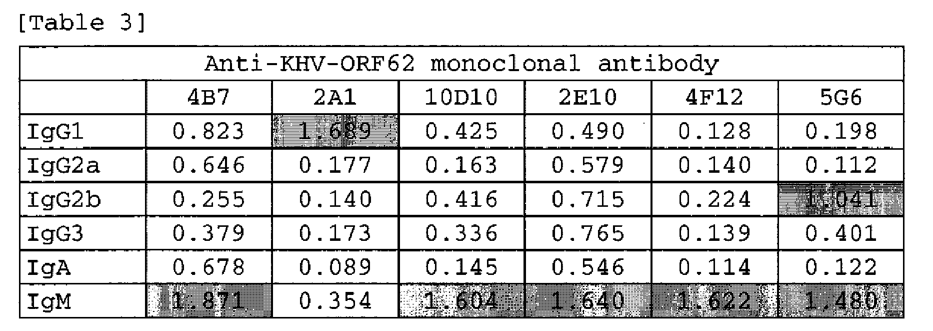

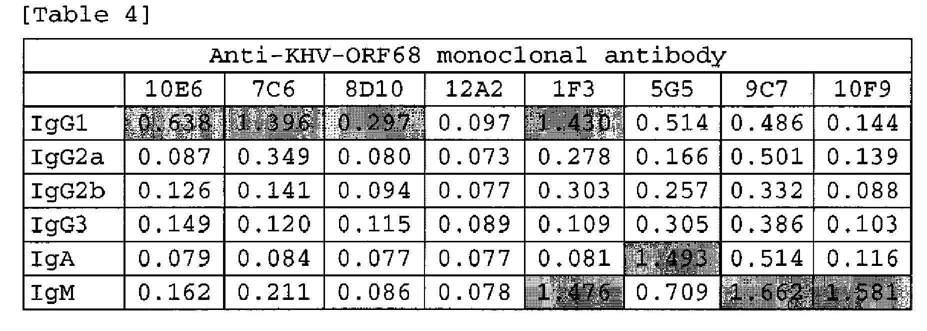

As a result, the anti-KHV-ORF62 mouse monoclonal antibody 10D10 was considered IgM. Moreover, the anti-KHV-ORF68 mouse monoclonal antibodies 7C6 and 10E6 were considered IgG1.

### 3-4. Confirmation of specificity of monoclonal antibody by Western blot

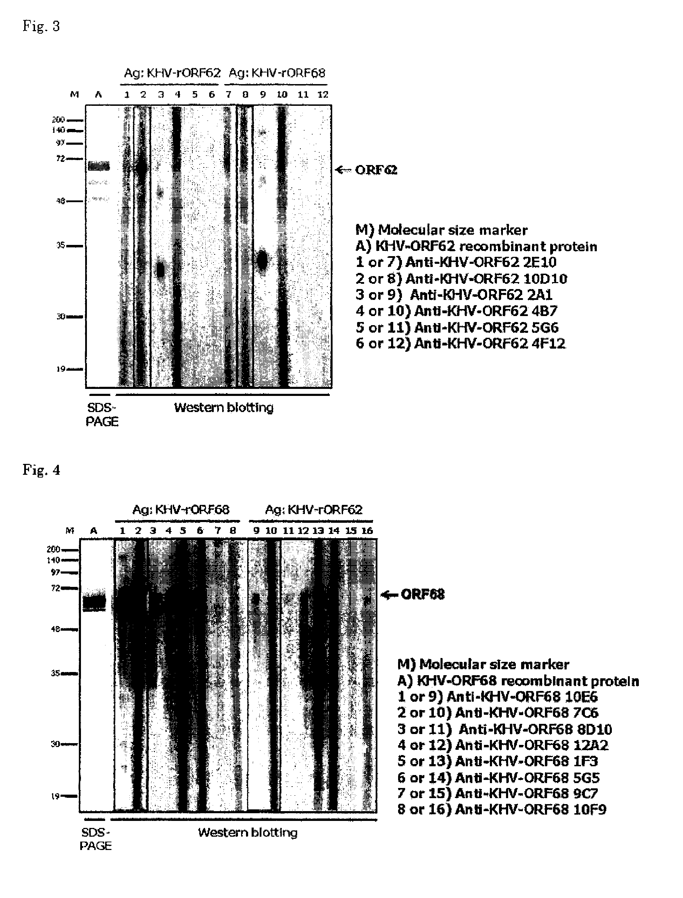

The obtained anti-KHV-ORF62 monoclonal antibody and anti-KHV-ORF68 monoclonal antibody were confirmed for their antigen specificity by Western blot analysis according to a routine method. The KHV-ORF62 antigen and the KHV-ORF68 antigen were subjected to SDS-PAGE (50 µg of each protein per lane) on a 10% polyacrylamide gel, and the proteins were then blotted onto a polyvinylidene fluoride (PVDF) membrane. Subsequently, the PVDF membrane was blocked for 45 minutes using 3% skimmed milk. The anti-KHV-ORF62 mouse monoclonal antibody and the anti-KHV-ORF68 mouse monoclonal antibody diluted with a TBS buffer were separately added to the membrane and shaken at room temperature for 2 hours. Subsequently, each monoclonal antibody was detected using an HRP-labeled anti-mouse IgG goat antibody (manufactured by Jackson ImmunoResearch Laboratories, Inc.) as a secondary antibody. Results of Western blot analysis on the specificity of the anti-KHV-ORF62 monoclonal antibody for the KHV-ORF62 antigen and the KHV-ORF68 antigen are shown in Figure 3. Results of Western blot analysis on the specificity of the anti-KHV-ORF68 monoclonal antibody for the KHV-ORF68 antigen and the KHV-ORF62 antigen are shown in Figure 4. The results shown in Figure 3 demonstrated that the anti-KHV-ORF62 mouse monoclonal antibody 10D10 specifically detected approximately 60 kDa KHV-ORF62 antigens but did not detect approximately 56 kDa KHV-ORF68 antigens. Moreover, the results shown in Figure 4 demonstrated that the anti-KHV-ORF68 mouse monoclonal antibodies 10E6 and 7C6 specifically detected approximately 56 kDa KHV-ORF68 antigens but did not detect approximately 60 kDa KHV-ORF62 antigens.

### 3-5. Detection of virus using monoclonal antibody (Western blot)

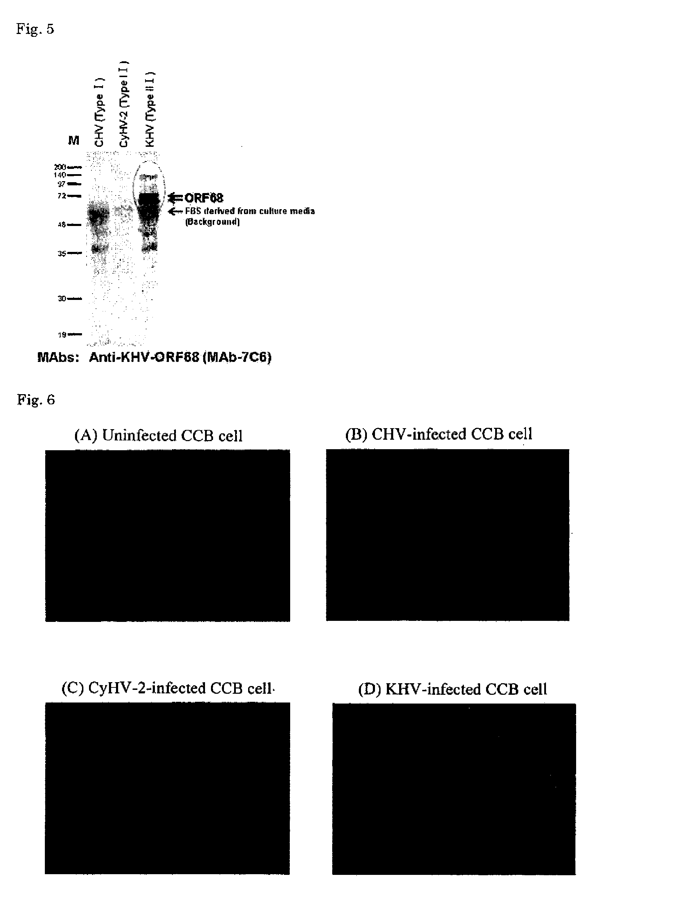

The anti-KHV-ORF62 mouse monoclonal antibody 10D10 thus confirmed not to exhibit cross-reactivity between the KHV-ORF62 antigen and the KHV-ORF68 antigen, and the anti-KHV-ORF68 mouse monoclonal antibodies 10E6 and 7C6 were used to detect viral antigens by Western blot for confirming whether or not the koi herpes virus was specifically detected. The sample used was a soluble fraction obtained by inoculating KF-1 cells separately with a koi herpes virus TUMST1 (KHV-TUMST1) strain, cyprinid herpes virus-1 (CHV), or cyprinid herpes virus-2 (CyHV-2), culturing the cells at 25°C for approximately 2 weeks, and then lysing the collected cells in a lysis buffer. The above samples prepared from the KHV-infected KF-1 cells, the CHV-infected KF-1 cells, and the CyHV-2-infected KF-1 cells were subjected to Western blot according to a routine method using each of the anti-KHV-ORF62 mouse monoclonal antibody 10D10 and the anti-KHV-ORF68 mouse monoclonal antibodies 10E6 and 7C6. Results about the anti-KHV-ORF68 mouse monoclonal antibody 7C6 are shown in Figure 5. A plurality of strong bands attributed to antigens probably formed by the processing of the protein encoded by the ORF68 sequence were confirmed for the KHV-infected KF-1 cells (right lane), whereas no such band was confirmed for the CHV-infected KF-1 cells (left lane) and the CyHV-2-infected KF-1 cells (central lane). It was confirmed that the band around 50 kDa in each lane was derived from fetal bovine serum (FBS) contained in the hybridoma culture solution. The results shown in Figure 5 demonstrated that the anti-KHV-ORF68 mouse monoclonal antibody 7C6 specifically detected koi herpes virus (KHV) but did not detect cyprinid herpes virus-1 (CHV) or cyprinid herpes virus-2 (CyHV-2). The hybridoma producing the anti-KHV-ORF68 mouse monoclonal antibody 7C6 was designated as a KHV hybridoma cell ORF68 (7C6) and deposited on March 24, 2010 with the National Institute of Technology and Evaluation, Patent Microorganisms Depositary (Accession No. NITE ABP-919).

### 3-6. Detection of virus using monoclonal antibody (fluorescent antibody method)

The anti-KHV-ORF68 mouse monoclonal antibody 7C6 was used to detect and confirm koi herpes virus in cells by a fluorescent antibody method. The sample used was prepared by culturing in advance CCB cells (common carp brain cells; carp brain-derived established cell line) using Lab-Tek chamber slide w/cover Permanox Slide sterile (manufactured by Nalge Nunc International Corp.), inoculating the cells with a koi herpes virus TUMST1 (KHV-TUMST1) strain, cyprinid herpes virus-1 (CHV), or cyprinid herpes virus-2 (CyHV-2), and culturing the cells at 20°C for 7 to 10 days. CCB cells uninfected with the virus were used as a control. The above samples prepared from the KHV-infected CCB cells, the CHV-infected CCB cells, and the CyHV-2-infected CCB cells were smeared over the slide and fixed with 100% methanol. Then, the slide was washed with PBS. Subsequently, a blocking solution (PBS-T; 50% skimmed milk, 0.05% Tween-20) was added to the smeared preparation and incubated at 37°C for 30 minutes. The anti-KHV-ORF68 mouse monoclonal antibody 7C6 was added thereto as a primary antibody and reacted at 37°C for 1 hour. Then, the slide was washed. Subsequently, an FITC-labeled anti-mouse IgG goat antibody (manufactured by Jackson ImmunoResearch Laboratories, Inc.) was added thereto as a secondary antibody and reacted at 37°C for 1 hour. Then, the slide was washed. A mounting agent (10% glycerol) was added to the slide, which was then observed using a fluorescence microscope. The results are shown in Figure 6. Strong fluorescence was observed in the KHV-infected CCB cells (D), compared with the uninfected CCB cells (A), the CHV-infected CCB cells (B), and the CyHV-2-infected CCB cells (C), demonstrating that use of the anti-KHV-ORF68 mouse monoclonal antibody 7C6 allowed specific detection of koi herpes virus in koi herpes virus-infected cells or tissues.

### Industrial Applicability

An antigenic peptide of the present invention is used for preparing a polyclonal or monoclonal antibody that is free from cross-reactivity and is specific for koi herpes virus. A monoclonal antibody produced by a hybridoma of the present invention is used in the specific detection of koi herpes virus. Moreover, a koi herpes virus test method of the present invention is useful in accurately diagnosing a history of infection in koi herpes virus-infected fish.

## Claims

1. A koi herpes virus-specific antigenic peptide consisting of the following peptide (A) or (B):
(A) a peptide consisting of the amino acid sequence represented by SEQ ID NO: 1 or 3; and
(B) a peptide consisting of an amino acid sequence having a deletion, substitution or addition of one or more amino acids in the amino acid sequence represented by SEQ ID NO: 1 or 3.

2. A koi herpes virus-specific antigenic peptide which is a partial peptide of a peptide consisting of the amino acid sequence represented by SEQ ID NO: 1 or 3 and comprises a koi herpes virus-specific antigenic determinant.

3. An anti-koi herpes virus-specific antibody which is obtained by immunization with a koi herpes virus-specific antigenic peptide according to claim 1 or 2 and specifically recognizes the koi herpes virus-specific antigenic peptide according to claim 1 or 2.

4. The anti-koi herpes virus-specific antibody according to claim 3, wherein the antibody is an anti-koi herpes virus-specific polyclonal antibody.

5. The anti-koi herpes virus-specific antibody according to claim 3, wherein the antibody is an anti-koi herpes virus-specific monoclonal antibody.

6. A hybridoma cell ORF68 (7C6) (NITE ABP-919) producing an anti-koi herpes virus-specific monoclonal antibody.

7. A diagnostic kit for koi herpes virus disease, comprising an antigenic peptide according to claim 1 or 2.

8. A detection kit for koi herpes virus, comprising the anti-koi herpes virus-specific antibody according to any one of claims 3 to 6.


SEQUENCE LISTING

<110>  National University Corporation Tokyo University of Marine Science
and Technology

<120>  KOI HERPES VIRUS (KHV)-SPECIFIC ANTIBODY AND ANTIGEN THEREOF

<130>  F2009-005

<150>  JP2009-077737
<151>  2009-03-26

<160>  8

<170>  PatentIn version 3.1

<210>  1
<211>  570
<212>  PRT
<213>  Artificial

<220>
<223>  KHV_ORF62

<400>  1

Asp Gln Ile Pro Pro Val Pro Ser Pro Thr Ser Ser Met Ala Ala Gly
1               5                   10                  15

Gly Gly Phe Glu Ile Pro Glu Gln Leu Gln Leu Glu Leu Ser Ser Leu
            20                  25                  30

Phe Gly Ala Asp Glu Leu Arg Thr Val Thr Tyr Arg Leu Arg Asn Arg
        35                  40                  45

Asp Val Phe Ala Thr Asp Ser Ser Thr Phe Val Pro Pro Leu Gln Gln
    50                  55                  60

Lys Gln Arg Gln Gln Gln Arg Ala Ser Ala Ala Pro Ser Thr Ala Gly
65                  70                  75                  80

Tyr Gln Ser Asn His Gly Leu Glu Pro Ser Thr Pro Asn Pro Pro Thr
                85                  90                  95

Ala Phe Thr Gln Gln Gln Pro Thr Ala Pro Arg Glu Asp Val Pro Ala
            100                 105                 110

Ala Arg Val Asp Leu Asn Val Val Ala Asn Ile Ala Ala Arg Asn Ser
        115                 120                 125

Arg Gln Gln Gln Asp Gln Gln Ala Glu Glu Glu Tyr Tyr Arg Gln Gln
    130                 135                 140

Glu Gln Glu Gln Arg Ala Ala Arg Glu Arg Leu Ala Ile Glu Gln Ala
145                 150                 155                 160

Glu Arg Glu Ala Arg Asp Arg Val Leu Gln Gln Gln Ala Ala Gln Ala
                165                 170                 175

Gln Glu Asp Glu Gln Gln Tyr Asn Glu Ser Asp Ser Ser Gln Asp Gly
            180                 185                 190

Gln Arg Val Pro Pro Pro Asp Asp Val Gln Thr Gly Ala Tyr Gly Tyr
        195                 200                 205

Arg Ala Pro Asn Met Tyr Gly Val Glu Tyr Ser Asn Gln Glu Glu Tyr
    210                 215                 220

Thr Arg Trp Tyr Pro Gln Tyr Ser Gln Asp Asp Tyr Gly Gln Gly Ser
225                 230                 235                 240

Gln Gln Asp Tyr Gly Gln Gly Ser Gln Gln Gln Ala Thr Glu Val Ile
                245                 250                 255

Val Gly Gln Val Ser Gly Gln Gly Ser Gly Gln Leu Thr Gly Thr Gly
            260                 265                 270

Asn Ile Phe Thr Leu Ala Asn Thr Gly Asn Thr Gly Asn Pro Gly Thr
        275                 280                 285

Leu Gly Asn Thr Asn Tyr Asn Gly Asn Gly Thr Ser Tyr Ile Asn Pro
    290                 295                 300

Thr Asn Pro Leu Asn Pro Thr Gly Pro Ile Arg Ile Thr Gly Thr Gly
305                 310                 315                 320

Leu Pro Gly Thr Gly Asn Ser Gly Thr Gly Asn Pro Gly Pro Leu Pro
                325                 330                 335

Pro Gly Asn Pro Gly Thr Thr Gly Asn Thr Gly Thr Gly Asn Thr Gly
            340                 345                 350

Asn Pro Gly Phe Ser Gly Asn Ser Gly Phe Ser Gly Asn Ser Gly Asn
        355                 360                 365

Pro Gly Pro Pro Pro Gly Pro Pro Gly Asn Pro Gly Gly Asn Ser Gly
    370                 375                 380

Gly Gly Gly Gly Ser Gly Gly Asn Pro Pro Pro Asn Pro Pro Pro Phe
385                 390                 395                 400

Gly Pro Pro Pro Gly Gly Arg Gly Phe Phe Gly Gly Gly Gly Ser Gly
                405                 410                 415

Ser Gly Gly Arg Gly Gly Gly Gly Gly Gly Gly Arg Gly Phe Asn Asn
            420                 425                 430

Gly Gly Ser Ala Leu Asn Arg Pro Arg Ser Ser Ala Arg Pro Val Asp
        435                 440                 445

Met Thr Gln His Ile Leu Ala Ser Asn Ser Asn Ile Ala Pro Phe His
    450                 455                 460

Asp Val Tyr Asp Glu Gln Leu Arg Ile Val Ile Leu Arg Asp Val Ser
465                 470                 475                 480

Ala His Leu Thr Gly Thr Val Ala Pro Arg Phe Arg Arg Val Pro Val
                485                 490                 495

Phe Val Pro Gln Pro Ala Gln Gln Gly Thr Leu Pro Gly Leu Phe Glu
            500                 505                 510

Pro Asn Leu Ala Tyr Met Gln Trp Tyr Asp Gly Ala Leu Asn Asn Ala
        515                 520                 525

Ala Ala Ala Asn Ile Val Tyr Pro Ala Leu Pro Asn Pro Ser His Phe
    530                 535                 540

Asp Gly Asn Pro Asn Pro Asp Ile Arg Phe Asp Val Ser Gln Glu Val
545                 550                 555                 560

Gln Glu Lys Phe Asp Ala Thr Ala Met Ile
                565                 570

<210>  2
<211>  1710
<212>  DNA
<213>  Artificial

<220>
<223>  KHV_ORF62

<400>  2
gatcagattc cccccgtccc atcacccacc agcagcatgg cggccggcgg cgggttcgag      60

atcccggagc aactgcagtt ggagctgagc agcctctttg gcgccgatga gctccggacc     120

gtgacctaca ggctccgcaa ccgcgacgtg tttgctaccg attcttcgac gttcgtgcct     180

cccctgcagc agaagcagag gcagcagcag cgggcctccg cagcgccctc cacggcgggg     240

taccaatcga atcacggcct cgagccctcg acgcccaacc cgccgactgc cttcacgcag     300

cagcagccga cggcgcccag agaagacgta cctgcggcgc gagtggacct caacgttgtg     360

gccaacatcg ccgccaggaa ctcgagacag cagcaggacc agcaggccga ggaggagtac     420

taccggcagc aggagcagga gcagcgcgcc gcccgggagc gtctggccat cgagcaggca     480

gagcgcgagg ccagagacag ggtcctccag cagcaggccg cgcaggcaca ggaggacgaa 540

cagcagtaca acgagtccga cagcagccag gatggtcaac gcgtgccgcc gcccgacgac 600

gtgcaaacgg gcgcgtacgg gtaccgtgcg ccgaacatgt acggggtcga gtacagcaac 660

caagaagaat acactcggtg gtatccgcag tattcgcaag acgactacgg ccagggctcc 720

cagcaagact acggccaggg ctcccagcag caagccaccg aagtcatcgt cggtcaagtg 780

tctggccagg gcagcgggca gctcacgggg accggcaaca tcttcacgct ggccaacacc 840

ggcaacactg gcaacccagg gacgctcggc aacaccaact acaacggcaa cggcacgtcg 900

tacatcaacc caaccaaccc cctcaacccg acggggccca tccgcatcac ggggaccggc 960

ctcccgggca cgggcaactc cgggacgggc aacccgggtc ctcttccacc cggcaaccca 1020

ggcaccacgg gcaacacagg gacaggcaac acgggtaacc caggcttctc tggcaactct 1080

ggcttcagcg gtaacagcgg caacccgggt ccaccacccg gaccgccagg caacccaggc 1140

ggcaacagcg gaggcggcgg cggcagtggc ggcaaccctc caccaaatcc accgccgttt 1200

ggtcctcctc caggcggacg cggatttttc ggcggcggcg gctcgggatc gggcggccgc 1260

ggcggaggcg gcggcggagg ccgaggcttc aacaacggcg ggtcggcgct gaaccgtccc 1320

cgctcctcgg ccaggcccgt ggacatgacc cagcacatcc tggccagcaa cagcaacatc 1380

gcgcccttcc acgacgtcta cgacgagcag ctccgcatcg taatcctccg cgacgtgagc 1440

gcgcacctga ccggcacggt cgcccctcgc ttcagacgcg tacccgtctt cgtgccgcag 1500

cccgcacagc agggcaccct gccgggtctc tttgagccca acctggccta catgcagtgg 1560

tacgacggcg cgctcaacaa cgccgcggcg gccaacatcg tctacccggc gctgcccaac 1620

ccctctcact ttgacggcaa ccccaacccc gacatccgct tcgacgtctc gcaggaggtg 1680

caggaaaagt ttgacgccac cgcgatgatc 1710

<210> 3
<211> 501
<212> PRT
<213> Artificial

<220>
<223> KHV_ORF68

<400> 3

Asp Gln Phe Lys Gln Thr Thr Ala Glu Leu Ala Gly Ile Lys Ala Asp
1               5                   10                  15

Ala Gln Ser Val Ala Asp Ala Ile Lys Ala Ala Asn Asp Glu His Gln
            20                  25                  30

Lys Thr Lys Asp Ala Leu Ala His Thr Gln Ser Ala Tyr Asp Leu Thr
        35                  40                  45

Met Glu Gln Val Asn Glu Tyr Gln Ala Lys Leu Gln Ala Ser Glu Ala
    50                  55                  60

Ala Leu Lys Glu Ala His Asp Thr Ala Lys Ala Asn Met Thr Val Lys
65                  70                  75                  80

Ala Gln Ser Arg Ile Ser Glu Leu Glu Ala Glu Ala Val Leu Gly Arg
                85                  90                  95

Asp Thr Met Lys Val Lys Asp Ala Glu Val Arg Asp Leu Lys Thr Lys
            100                 105                 110

Leu Ala Ser Ala Lys Asn Glu Thr Ala Ala Ala Leu Ser Arg Leu Ala
        115                 120                 125

Asp Lys Glu Asp Glu Ile Glu Thr Leu Ala Lys Thr Leu Ala Asp Ala
    130                 135                 140

Val Glu Ser Ala Lys Gln Gln Asn Glu Thr Ile Gly Arg Asn Ala Ala
145                 150                 155                 160

Glu Ser Ile Lys Asp Leu Ala Leu Arg Leu Lys Arg Gln Glu Glu Leu
                165                 170                 175

Ala Lys Asp Ala Glu Ala Asn Leu Ala Asn Val Thr Arg Asp Leu Glu
            180                 185                 190

Arg Arg Met Lys Glu Gln Ala Glu Glu Tyr Asn Asn Asn Glu Leu Asn
        195                 200                 205

Tyr Ala Ala Ala Leu Thr Ala Leu Gln Gln Ser Ser Lys Glu Leu Glu
    210                 215                 220

Gln Gln Leu Ala Asp Ala Arg Arg Gln Leu Ala Gln Ala Gln Ala Gln
225                 230                 235                 240

Ala Gln Ala Gln Ala Ala Gln Pro Gln Pro Gln Pro Pro Ala Gln Pro
                245                 250                 255

Gln Ala Ala Ala Gln Pro Pro Ala Gln Pro Gln Ala Ala Gln Ala Pro
            260                 265                 270

Ala Gln Pro Gln Pro Pro Ala Ala Gln Pro Gln Ala Gln Ala Ala Ala
        275                 280                 285

Val Ile Ala Gln Gly Val Thr Glu Ala Ala Ser Val Ala Asp Asp Gly
    290                 295                 300

Glu Thr Pro Met Glu Glu Ile Glu Pro Pro Glu Tyr Glu Pro Pro Ala

305                 310                 315                 320

Gly Gly Val Glu Tyr Glu Pro Asp Met Glu Ile Glu Met Pro Pro Ala
                325                 330                 335

Ala Phe Asp Pro Glu Glu Tyr Arg Lys Ser Gln Glu Arg Lys Lys Leu
            340                 345                 350

Glu Glu Ser Ala Arg Ala Asn Pro Asn Leu Val Arg Lys Pro Thr Pro
        355                 360                 365

Lys Val Asn Met Phe Asp Phe Met Gln Ser Ile Lys Lys Pro Val Pro
    370                 375                 380

Glu Thr Val Glu Ala Leu Lys Ala Arg Val Lys Glu Leu Glu Met Met
385                 390                 395                 400

Leu Lys Leu Val Tyr Asp Glu Leu Thr Arg Val Asn Ala Glu Arg Tyr
                405                 410                 415

Gln Glu Ala Ala Thr Arg Cys Met Ala Ala Glu Asn Phe Lys Arg Gln
            420                 425                 430

Ile Lys Asp Leu Arg Ser Lys Asn Val Ala Leu Arg Asp Lys Leu Glu
        435                 440                 445

Glu Ala Arg Leu Arg Phe Glu Leu Asp Lys Leu Thr Tyr Glu Asn Asp
    450                 455                 460

Tyr Ala Lys Gln Met Tyr Lys Phe Gln Glu Asn Ile Lys Val Glu Tyr
465                 470                 475                 480

Lys Gly Ser Val Asp Met Val Asp Gln Leu Lys Arg Glu Asn Ser Arg
                485                 490                 495

Leu Glu Ser Gln Ile
            500

<210>  4
<211>  1503
<212>  DNA
<213>  Artificial

<220>
<223>  KHV_ORF68

<400>  4
gatcagttca agcagaccac ggccgagctg gccggcatca aggccgacgc tcagagcgtc      60

gccgacgcca tcaaggcggc gaacgacgag caccagaaga ccaaggatgc cctggcccac     120

acgcagagcg cctacgacct gaccatggag caggtcaacg agtaccaggc caagctccag     180

gccagcgagg ccgcgctcaa ggaggcgcac gacacggcca aggccaacat gaccgtcaag 240

gcccagtcgc gcatctccga gctcgaggcc gaggccgtgc tgggccgcga caccatgaag 300

gtcaaggacg ccgaggtgcg ggacctcaag accaagctgg ccagcgccaa gaacgagacg 360

gccgcagcgc tcagccgcct cgcggacaag gaagacgaga tcgagacgct ggccaagacg 420

ctcgccgacg ccgtcgagag cgccaagcag cagaacgaga ccatcggccg caacgccgcc 480

gagtccatca aggacctggc gctcaggctc aagcgccagg aggagctcgc caaggacgcc 540

gaggccaacc tcgcgaacgt gacccgggac ctcgagcggc gcatgaagga gcaggccgag 600

gagtacaaca acaacgagct caactacgcc gccgccctca ccgccttgca gcagtcgtcc 660

aaggaactgg agcaacagct ggccgacgcg cgacgccagc tcgcccaggc ccaggcccag 720

gcccaggccc aggccgctca acctcaaccc cagccacccg ctcaacccca ggcggccgct 780

cagccacccg ctcaacccca ggccgctcag gcacccgctc aaccccagcc acccgccgct 840

caaccccaag cgcaggcggc ggcggtcatc gcgcaaggcg tcactgaggc cgcctccgtc 900

gcggacgatg gcgagacgcc catggaggag attgagccgc ccgagtacga gcctccggcc 960

ggcggcgtcg agtacgagcc cgacatggag atcgagatgc cgcccgccgc cttcgacccc 1020

gaagagtacc gcaagtccca ggagcgcaag aagctcgagg agagcgcccg cgccaacccc 1080

aacctcgtgc gcaagccgac tcccaaggtc aacatgttcg actttatgca gtccatcaag 1140

aagccggtgc ccgagacggt ggaggccctc aaggcccggg tgaaggagct ggagatgatg 1200

ctgaaactgg tgtacgatga gctcacgcgg gtcaacgccg agcgctacca ggaggcggcc 1260

acccgctgca tggcggccga gaacttcaag cgccagatca aggacctgcg ctccaagaac 1320

gtcgcgctca gggacaagct cgaggaggcc cggctccgct tcgagctgga caagctcacc 1380

tacgagaacg actacgccaa gcagatgtac aagttccagg agaacatcaa ggtcgagtac 1440

aagggcagcg tggacatggt ggaccagctg aagcgcgaga actccaggct cgagtcgcag 1500

atc 1503

<210> 5
<211> 36
<212> DNA
<213> Artificial

<220>
<223> primer KHV_62expF

<400> 5
aaggatccca tatggatcag attccccccg tcccat 36

<210> 6
<211> 32
<212> DNA
<213> Artificial

<220>
<223>  primer KHV_62expR

<400>  6
ttgaattctc acatcgcggt ggcgtcaaac tt                                    32


<210>  7
<211>  36
<212>  DNA
<213>  Artificial

<220>
<223>  primer KHV_68expF

<400>  7
aaggatccca tatggatcag ttcaagcaga ccacgg                                36


<210>  8
<211>  32
<212>  DNA
<213>  Artificial

<220>
<223>  primer KHV_68expR

<400>  8
ttgaattctc actgcgactc gagcctggag tt                                    32